# EUROPEAN PATENT APPLICATION

(11) **EP 3 366 195 A1**
(43) Date of publication of application: **29.08.2018**
(21) Application number: 17157494.0
(22) Date of filing: 22.02.2017
(51) Int. Cl.: A61B 5/00, G06T 7/00, B26B 19/38, B26B 21/40, A45D 44/00, G06K 9/00

(54) **SYSTEM AND METHOD FOR DETECTING SKIN CONDITIONS**

(71) Applicant: Koninklijke Philips N.V., 5656 AE Eindhoven (NL)
(72) Inventor: MENA BENITO, Maria Estrella, 5656 AE Eindhoven (NL); KIRENKO, Ihor Olehovych, 5656 AE Eindhoven (NL)
(74) Representative: de Haan, Poul Erik

(57) **Abstract**

The present disclosure relates to a system (60) for remotely detecting a skin condition, particularly skin irritations, the system (60) comprising a sensing unit (30) comprising at least one image sensor (32, 34) arranged to remotely monitor a skin portion of a user (62) while performing a personal care procedure with a personal care appliance (40), a control unit (70) arranged to process image data comprising skin condition information that is indicative of skin conditions, wherein the image data is provided by the sensing unit (30), wherein the control unit (70) is further arranged to correlate position data of the personal care appliance (40) with the skin condition information, and wherein the control unit (70) is arranged to provide control commands. The disclosure further relates to a method of remotely detecting a skin condition, particularly skin irritations, and to a method of operating a personal care appliance (40).

## Description

### FIELD OF THE INVENTION

The present disclosure relates to a system for remotely detecting a skin condition, particularly skin irritations, that is operable and useful in the context of a personal care procedure. More particularly, in the context of the present disclosure, a personal care procedure involves a use of a personal care appliance, such as a grooming appliance, a hair cutting appliance, a shaving appliance, etc.

The present disclosure further relates to a corresponding method of remotely detecting a skin condition, particularly skin irritations. In addition, the present disclosure relates to a related method of operating a personal care appliance.

As used herein, a personal care procedure is frequently referred to as a shaving procedure. This shall, however, not be understood in a limiting sense.

### BACKGROUND OF THE INVENTION

US 2014/0137883 A1 discloses a razor and a method of controlling a razor, the razor comprising at least one blade, and an imaging device that is coupled to the razor, wherein further a processor is provided that is configured to detect a state in which the razor is adjacent to the skin of a user of the razor, to determine, via the imaging device, whether an area of the skin is sensitive to shaving, and, in case it is determined that a certain area of the skin is sensitive to shaving, to operate a protective device that is coupled to the razor to adjust a position of the blade of the razor with respect to the skin.

For many individuals, particularly for male users, shaving is one of the most common personal care procedures. For many individuals, shaving forms part of the daily routine.

A general goal of frequent or even daily shaving is to have a well-groomed appearance and a smooth skin, particularly a smooth facial skin. Hence, a preferably clean and close shave is desired. To this end, shaving appliances and razors are preferably equipped with sharp blades. In this way, particularly facial hair can be cut or chopped close to the skin. This, however, involves a certain risk of skin irritations.

Skin irritations occur both in manual shaving using a razor and in motor-powered shaving using a shaving appliance. Further, skin irritations occur both in the context of wet shaving and dry shaving.

The term skin irritation covers a group of phenomena. For instance, ingrown hairs may be present when a hair becomes trapped beneath the skin surface. This may give rise to local inflammation, lumps and pimples.

Further, so-called shaving bumps result as an undesirable side effect of a shaving procedure. In addition, so-called shaving rush may be present involving reddened skin portions, typically at the neck. A so-called razor burn has a similar effect, involving a reddened skin and a hot and itchy sensation at the skin.

Often, the above phenomena are somewhat interrelated. Generally, skin irritations are caused and intensified by at least one of performing a dry shave rather than a wet shave, using a blunt blade, having a generally sensitive skin, and performing the shave in an unconditioned state of the skin (i.e. no use of shaving foam, shaving cream, and such like).

There are several approaches to and recommendations for reducing the risk of skin irritations in connection with shaving. However, skin irritations are still experienced by many users in their daily grooming routine.

It has been observed that, on the one hand, skin irritations are clearly visible, when the skin is in a reddened state. However, on the other hand, it has been observed that emerging skin irritations are, in an initial state, not clearly visible to the human eye. This makes the prediction and detection of an increased risk of skin irritations problematic.

Further, severe skin irritations often occur when an already slightly irritated and generally sensitive skin portion is subjected to an intense and acute shaving procedure.

Hence, it would be beneficial to detect and, in some respect, predict an increased risk of skin irritations in respective skin regions. If this was the case, appropriate corrective action may be taken.

Hence, it would be beneficial to have further in-depth information about a present skin condition, preferably at a moment where a further deterioration of the skin state may still be avoided by appropriate counter measures.

### SUMMARY OF THE INVENTION

It is an object of the present disclosure to provide a system for detecting a skin condition that is operable in a personal care environment. Preferably, the system is operable to detect a current skin condition and to control the operation of an involved personal care appliance accordingly. Preferably, the system provides for an improved shaving procedure involving information about a current state of the processed skin. As a consequence, it is desirable that the system is capable of detecting emerging or imminent skin irritations which may be worsened when an intense personal care procedure, for instance a shaving procedure, takes place.

Hence, it would be beneficial to provide a system that is capable of early warning of skin irritations and of operating the personal care appliance accordingly. Preferably, the system provides extended diagnosis capabilities, beyond a mere visual detection of irritations or a reddening of the skin.

It is a further object of the present invention to provide a corresponding method of detecting a skin condition and a method of operating a personal care appliance under consideration of detected skin condition information.

In a first aspect of the present disclosure a system for remotely detecting a skin condition, particularly skin irritations, is presented, the system comprising:
a sensing unit comprising at least one image sensor arranged to remotely monitor a skin portion of a user while performing a personal care procedure with a personal care appliance,
a control unit arranged to process image data comprising skin condition information that is indicative of skin conditions,
wherein the image data is provided by the sensing unit,
wherein the control unit is further arranged to correlate position data of the personal care appliance with the skin condition information, and
wherein the control unit is arranged to provide control commands.

This aspect is based in the insight that a remote sensing unit may be provided that is, on the one hand, arranged to monitor a considerably large portion of the user, for instance the whole face and neck portion. Hence, in contrast to sensing arrangements that are attached to the personal care appliance, a remotely operating system is provided that is capable of detecting skin conditions in regions where the personal care appliance is not necessarily present at the very moment.

In other words, the sensing unit may detect a skin condition of a skin portion before the personal care appliance contacts or engages this region. Since the system is further arranged to correlate position data of the personal care appliance with the skin condition information, it is possible to provide a forecast, and to identify skin regions that are potentially vulnerable to more severe skin injuries and irritations. As a consequence, an early detection of skin irritations is possible which enables appropriate counter measures to be taken.

Further, in at least some embodiments, a real time or nearly real time skin monitoring is enabled. Hence, the user may be guided through the personal care procedure to improve the overall result, and to significantly reduce accompanying irritations and damages. In this way, the general condition and health of the skin may be improved as the likelihood of severe skin irritations due to the personal care procedure is greatly reduced, both on a short-term and a long-term scale.

Further, as the control unit is, in at least some embodiments, operatively coupled with the personal care appliance, control commands may be sent to the appliance. This may involve, in some embodiments, a reduction of an intensity level of the personal care procedure. Further, in some embodiments, counter measures may involve prompting a user to avoid highly sensitive regions and/or to process potentially susceptible regions in a gentle and careful fashion.

A further benefit of the system is that premature aging of the skin, due to skin irritations frequently occurring in personal care procedures, may be avoided or, at least, delayed.

In one exemplary embodiment, the sensing unit is arranged to detect and the control unit is arranged to process image data in the visible range and in the non-visible range. As used herein, the visible range covers a portion of the electromagnetic spectrum that is typically visible to the human eye. Hence, the visible range may for instance involve a range from about 390 nm (nanometers) to about 700 nm. Adjacent to the visible range, the infrared range and the ultraviolet range is provided. Infrared radiation covers radiation having longer wavelengths than those of the visible light. Depending on the respective definition, infrared light may cover the range between about 700 nm and about 1,000 µm (micrometer). In practice, primarily a so-called near-infrared radiation is used which may cover, depending on the definition, a range between about 700 nm and 1,400 nm.

Ultraviolet radiation is electromagnetic radiation having shorter wavelengths than those of visible light and may cover a range of about 10 nm to about 390 nm. Needless to say, there is no sharp border between the respective regions of the electromagnetic radiation.

Providing the sensing unit and the control unit with the capability of processing non-visible radiation has the advantage that skin condition indicative information may be detected that is not clearly visible to the human eye. Hence, emerging irritations may already be detected in an initial state.

A general working principle of the system is that skin irritations basically evoke or trigger a dermal repair process that requires additional nutrition. This process causes the reddening of the skin in the vicinity of the initial irritation.

In some individuals, permanent or temporary skin defects are already present before the personal care procedure actually takes place. This may for instance involve the presence of scars, sunburns, freckles, birth marks, warts, and such like. Further, temporary irritations, involving sunburns, incisions, and such like may be present. It may be therefore beneficial to utilize not only a visible radiation portion but also invisible radiation portions as in this way a greater penetration depth may be present.

In one exemplary embodiment, the sensing unit, particularly the at least one light source, is integrated in or disposed adjacent to a mirror, for instance a bathroom mirror. This has the advantage that in an everyday setting where a personal care procedure is performed, the sensing equipment may be provided which has no negative impact or adverse influence on the usual personal care practice. Further, in front of a mirror, typically a sufficient lighting situation is present so that also the remotely operating sensing unit may detect slight and minute changes of blood perfusion and, more generally, of the face color or complexion.

In another exemplary embodiment, the sensing unit and the control unit form a remote photoplethysmography device, wherein the sensing unit is a camera based non-contact sensing unit. Hence, the sensing unit involves at least one image sensor that implements CCD-elements, CMOS-elements, and such like. Depending on the spectral responsivity of the at least one image sensor, respective portions of the visible range and, in at least some embodiments, of the invisible range may be covered.

In some embodiments, image information provided by the sensing unit involves a first emphasis at the red portion of the electromagnetic radiation and a second emphasis of the near infrared portion of the electromagnetic radiation. Hence, two ranges of the electromagnetic radiation may be provided and processed in which a different absorption and reflection behavior is provided in the observed tissue.

Arranging the sensing unit as a remote photoplethysmography sensing unit extends the processing capability beyond limited image processing of images in the visible range of electromagnetic radiation. In this way, more information about potential skin irritations and a susceptibility of skin portions to irritations may be obtained.

In a further exemplary embodiment, the sensing unit is further capable of detecting the current position of the personal care appliance in an observed region. To this end, image processing algorithms for object tracking (video tracking) over time in the captured image data may be used.

In a further exemplary embodiment, a plurality of image sensors is provided that are spaced from one another. For instance, the sensing unit may comprise at least two cameras that are arranged at a distance in the vicinity of a bathroom mirror. Having two or more image sensors also allows for a multi-dimensional (three-dimensional or stereo) observation of the user. This may have a positive effect on the tracking of the personal care appliance. Further, using stereo image data or three-dimensional image data may simplify the detection and mapping of skin regions, for instance to distinguish the face from the neck, the cheeks from the nose, etc.

In a further exemplary embodiment, the system further comprises a personal care appliance, particularly a hand-wearable personal care appliance, wherein the control unit is operatively coupled with the personal care appliance. In this way, the personal care appliance may be provided with control commands, thereby enabling an instant or nearly instant reaction to detected potentially irritation-prone skin conditions.

In a further exemplary embodiment, the personal care appliance is a grooming appliance, particularly a hair cutting appliance, that is operable to contact a skin region of a user that is monitored by the sensing unit. Hence, the personal care appliance may be arranged as a shaving appliance, for instance. Further, the personal care appliance may be arranged as a trimming appliance.

In an exemplary set-up, the system combines at least one camera that is integrated in the bathroom mirror or arranged adjacent thereto. Preferably, at least one camera is provided that is capable of PPG monitoring. As indicated above, in some embodiments, two or even more cameras may be provided to provide stereo or multi-dimensional image information.

The system in accordance with this set-up further comprises an electrical shaver or an electrical hair trimming appliance. Further, a control unit is provided. Generally, the control unit may be arranged at the side of the cameras. In the alternative, the control unit may be arranged on the side of the personal care appliance. Further, a distributed arrangement of the control unit may be envisaged. In yet a further conceivable embodiment, a separate control unit is provided, for instance a mobile computer, a mobile phone, a tablet computer, or a similar mobile computing device.

In either case, a communication between the sensing unit and the personal care appliance is established insofar as the control unit is supplied with image data and arranged to process the image data. Further, the control unit is arranged to provide control commands that are transmitted to the personal care appliance.

In accordance with exemplary aspects of the present disclosure, real-time or nearly real-time information of the skin state before, during, and after the personal care procedure is provided. Consequently, real-time or nearly real-time user guidance through the personal care procedure is possible. Hence, as with the exemplary shaving procedure, the likelihood of skin irritations and razor bums is greatly reduced.

Generally, continuous monitoring, quasi-continuous monitoring and/or spot-check monitoring of the skin state in reaction to the personal care appliance is possible.

Further, a comparison of an initial state before the personal care procedure and a final state of the skin after the personal care procedure is enabled. In this way, not only an instant or quasi-instant monitoring but also a mid-term to long-term monitoring is possible, e.g. on a day by day, a weekly or a monthly basis.

As an alternative to a plurality of image sensors, a single 3D-camera or stereo-camera may be used. Whenever a three-dimensional or stereo imaging is possible, shape-detection may be performed to map the face of the user and to correlate the position of the personal care appliance with the actual face shape.

A further benefit of a combined position tracking and skin imaging is that the system may detect and (virtually) plot a processing path and/or a map of processed regions and regions that have not been processed yet. In this way, additional user guidance may be provided, for instance to avoid double-processing of potentially sensitive regions and/or to ensure that each portion is at least once processed.

In a further exemplary embodiment, the control unit is arranged to control an operation condition of the personal care appliance in reaction to a detected skin condition state. Hence, no user intervention is required. In other words, the personal care appliance may automatically be adapted to an irritation-prone skin condition.

In a further exemplary embodiment, the control unit is arranged to adapt an intensity parameter of the personal care appliance. Hence, when a considerably robust and healthy skin portion is detected, a high intensity level is possible. By contrast, when a rather sensitive and vulnerable portion is detected, a low intensity level may be applied.

In a further exemplary embodiment, the intensity parameter is one of a blade operation speed of the personal care appliance and a working angle of the personal care appliance with respect to the skin.

The working angle may involve a blade working angle. Further, in some embodiments, the intensity parameter is an effective cutting length and/or the presence of guard features, etc.

In some exemplary embodiments, the intensity parameter is the discharge of a skin calming or soothing fluid. In another embodiment, the intensity parameter is an applied contact pressure.

In a further exemplary embodiment, the control unit is arranged to provide user feedback commands in reaction to the detected skin condition state. In this way, the system may prompt the user to perform counter actions which may involve, for instance, reducing a sliding speed and/or a blade operation speed of the personal care appliance, reducing a contact pressure, and/or avoiding a certain region.

User feedback commands may be provided as visual commands, audio commands, tactile commands, etc.

Further, in some exemplary embodiments, the system comprises an illumination unit comprising at least one light source. Preferably, the at least one light source is adapted to the spectral sensitivity of at least one image sensor of the sensing unit.

In still a further embodiment, the skin condition information is based on at least one of skin color detection and blood circulation detection. Preferably, a combination of skin color and blood circulation is used to assess the skin condition.

In a further exemplary embodiment, the control unit is arranged to detect temporal changes of the skin condition. In this way, a time behavior of the skin condition may be monitored and recorded. Hence, short-term, mid-term and long-term observations may be provided. In some embodiments, the control unit is arranged to compare an actual skin condition with a reference skin condition. The reference skin condition may be an initial skin condition prior to a personal care procedure. Further, the reference skin condition may be the skin condition of a previous day, week or month, for instance.

In a further aspect of the present disclosure, there is presented a method of remotely detecting a skin condition, particularly skin irritations, the method comprising:
- providing a sensing unit comprising at least one image sensor arranged to remotely monitor a skin portion of a user while performing a personal care procedure with a personal care appliance,
- providing a control unit,
- operating the control unit to process image data that is indicative of skin conditions, wherein the image data is provided by the sensing unit,
- deriving skin condition information from the image data, and
- operating the control unit to correlate position data of the personal care appliance with the skin condition information.

In a further aspect of the present disclosure there is presented a method of operating a personal care appliance, particularly a grooming appliance, the method comprising:
- detecting skin condition information in accordance with at least one embodiment of the detection method as disclosed herein,
- providing control commands for the personal care appliance, and
- operating the personal care appliance depending on the skin condition information by the control commands.

In still another aspect of the present disclosure, there is presented a computer program comprising program code means for causing a system in accordance with the present disclosure to carry out the steps of the operating method as described herein when said computer program is carried out on a computing device that is operatively coupled with or forms part of the skin condition detection system.

Preferred embodiments of the disclosure are defined in the dependent claims. It shall be understood that the claimed method has similar and/or identical preferred embodiments as the claimed system and as defined in the dependent claims.

### BRIEF DESCRIPTION OF THE DRAWINGS

These and other aspects of the invention will be apparent from and elucidated with reference to the embodiments described hereinafter. In the following drawings
Fig. 1 shows a simplified schematic perspective view of an exemplary setting of a personal care system implemented in a bathroom;
Fig. 2 is a perspective view of an exemplary embodiment of a personal care appliance that is arranged as a hair cutting appliance;
Fig. 3 is a simplified schematic view of an exemplary embodiment of a personal care monitoring system;
Fig. 4 is a further schematic simplified view of another exemplary embodiment of a personal care monitoring system; and
Fig. 5 is a simplified schematic block diagram illustrating an embodiment of a method of remotely detecting a skin condition and operating a personal care appliance.

### DETAILED DESCRIPTION OF THE INVENTION

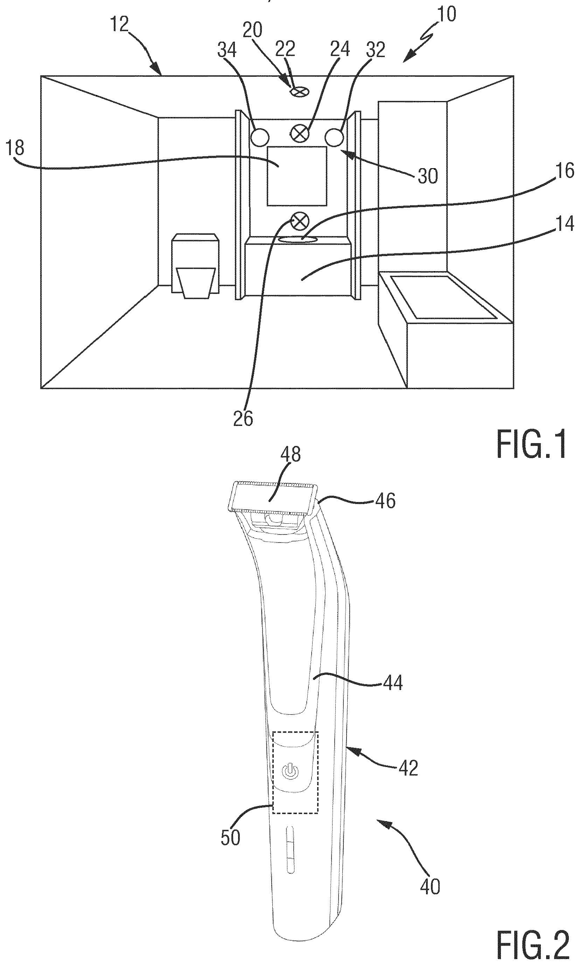

Fig. 1 is a schematic perspective view of an exemplary personal care setting 10 which is provided in a bathroom 12. In the bathroom 12, a washbasin 14 is present that includes a sink 16. Above the sink, a mirror 18 is present which may also be referred to as bathroom mirror. A user may assume a position in front of the mirror 18 and then perform a personal care treatment, for instance a shaving operation.

A personal care procedure typically involves handling a movable hand-guided appliance. This may involve approaching and contacting processing zones at the user, involving the facial region, the neck region, the chin region, the hair region/scalp region, further body parts, etc. Hence, an adequate illumination of the processing zone is appreciated to facilitate handling the appliance.

An illumination unit is indicated in Fig. 1 by 20. The illumination unit 20 involves a top or ceiling light source 22 that is arranged at a ceiling in front of the mirror 18. Further, a top light source 24 is provided that is arranged at the wall, adjacent to the mirror 18. In addition, a bottom light source 26 is provided that is arranged at a bottom end of the mirror 18. In addition, side light sources, etc. may be present.

The personal care setting 10 further involves a sensing unit 30 involving a first sensor 32 and a second sensor 34. Generally, the sensing unit 30 includes at least one image sensor 32, 34. The sensors 32, 34 in the exemplary arrangement of Fig. 1 are spaced away from one another and arranged in the vicinity of the mirror 18. In some exemplary embodiments, a so-called smart mirror is provided that implements at least one of a light source and an image sensor. In a smart mirror, the light source and/or the image sensor are not or only barely visible from the front of the mirror.

The at least one image sensor 32, 34 of the sensing unit 30 may involve a camera including a pattern of CCD-elements, CMOS-elements, etc.

Preferably, the at least one sensor 32, 34 is capable of detecting electromagnetic radiation both in the visible range and in the invisible range.

In Fig. 2, a personal care appliance 40 is shown. In exemplary embodiments, the personal care appliance 40 is arranged as grooming appliance, such as a hair trimmer, a shaver, and/or a combined trimming and shaving appliance. The personal care appliance 40 in Fig. 2 is arranged as a hair cutting appliance 42 that is also capable of shaving.

The hair cutting appliance 42 includes a housing 44 in which a motor, a drive train, a battery, controls, etc. are arranged. At a top end of the housing 44, a processing head 46 is provided. A blade set 48 is mounted to the processing head 46. The hair cutting appliance 42 is arranged to drive the blade set 48 to cut hair, particularly to shave hair. The housing 44 is arranged in an elongated fashion. The housing 44 of the hair cutting appliance 42 is arranged to be grabbed by the user and to be manually guided to perform a personal care procedure. Further types and arrangements of hair cutting appliances are conceivable.

The personal care appliance 40 further comprises an operation control unit 50 that controls the operation thereof. The operation control unit 50 may be operatively coupled with control elements, such as switches, indicators, and such like.

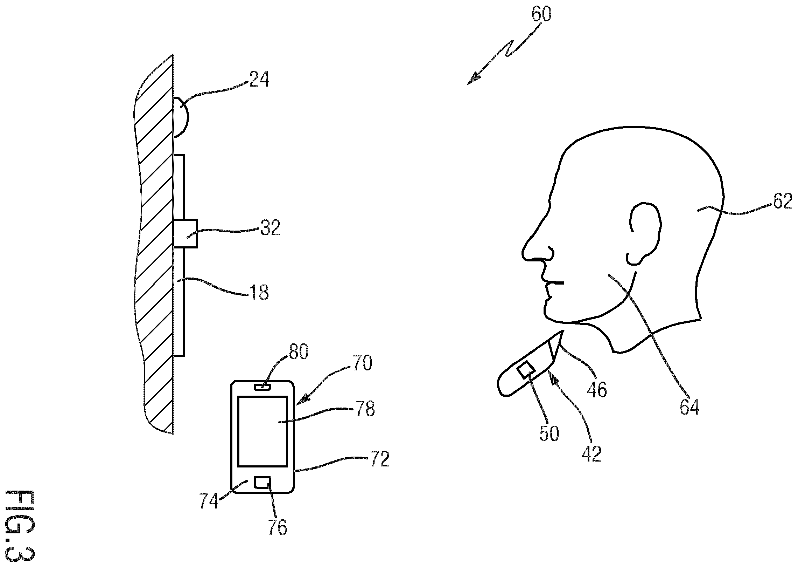

Fig. 3 is a schematic simplified lateral view of an operation control system 60 for a personal care procedure. In Fig. 3, a user 62 whose face is to be shaved is shown. Hence, a skin portion 64 of the user 62 is engaged and processed by a hair cutting appliance 42 having a processing head 46. As a side effect, this may cause skin irritations.

As already indicated in Fig. 1, the user 62 uses a mirror 18 to facilitate the operation and control of the hair cutting appliance 42. Adjacent to the mirror 18, a light source 24 and an image sensor 32 are present. Preferably, the light source 24 and the image sensor 32 are capable of PPG (photoplethysmographic) monitoring. Hence, skin color, perfusion and similar information may be remotely detected when observing the skin portion 64 of the user 62.

Quite recently, photoplethysmographic (PPG) systems that operate on a remote basis have been introduced. Generally, PPG systems are used in the field of vital signs detection. Vital signs of a person, for example the heart rate (HR), the respiration rate (RR) or the blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heartbeat affect transmission or reflectance correspondingly. Besides information about the heart rate, a PPG waveform can comprise information attributable to further physiological phenomena such as the respiration. By evaluating the transmissivity and/or reflectivity at different wavelengths (typically red and infrared), the blood oxygen saturation can be determined.

Conventional pulse oximeters for measuring the heart rate and the (arterial) blood oxygen saturation (also called SpO2) of a subject are attached to the skin of the subject, for instance to a fingertip, earlobe or forehead. Therefore, they are referred to as 'contact' PPG devices. A typical pulse oximeter comprises a red LED and an infrared LED as light sources and one photodiode for detecting light that has been transmitted through patient tissue. Commercially available pulse oximeters quickly switch between measurements at a red and an infrared wavelength and thereby measure the transmissivity of the same area or volume of tissue at two different wavelengths. This is referred to as time-division-multiplexing. The transmissivity over time at each wavelength gives the PPG waveforms for red and infrared wavelengths. Although contact PPG is regarded as a basically non-invasive technique, contact PPG measurement is often experienced as being unpleasant, since the pulse oximeter is directly attached to the subject and any cables limit the freedom to move.

Quite recently, also non-contact, remote PPG (R-PPG) devices for unobtrusive measurements that are operable in home user environments have been introduced. Remote PPG is a non-invasive technique which measures the small changes in color under the skin epidermis, caused by variations in volume and oxygen saturation of the blood in the vessels, due to heart beats.

Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

Verkruysse et al., "Remote plethysmographic imaging using ambient light", Optics Express, 16 (26), 22 Dec. 2008, pp. 21434-21445 demonstrates that photoplethysmographic signals can be measured remotely using ambient light and a conventional consumer level video camera.

Wieringa, et al., "Contactless Multiple Wavelength Photoplethysmographic Imaging: A First Step Toward "SpO2 Camera" Technology," Ann. Biomed. Eng. 33, 1034-1041 (2005), discloses a remote PPG system for contactless imaging of arterial oxygen saturation in tissue based upon the measurement of plethysmographic signals at different wavelengths. The system comprises a monochrome CMOS-camera and a light source with LEDs of three different wavelengths. The camera sequentially acquires three movies of the subject at the three different wavelengths. The pulse rate can be determined from a movie at a single wavelength, whereas at least two movies at different wavelengths are required for determining the oxygen saturation.

Using remote PPG technology, vital signs can be measured from video camera signals providing a time sequence of image frames, as it is revealed by minute light absorption changes in the skin caused by the pulsating blood volume.

An example for an extended application of PPG techniques is disclosed in WO 2016/096591 A1 that relates to a method and a corresponding device for use in allergy testing, the method comprising:
- receiving a first set of spatially distributed light intensity values covering a skin region of the subject including a location at which the substance has been applied, wherein the light intensity values in the first set are intensities of visible light,
- receiving a second set of spatially distributed light intensity values covering the skin region, wherein the light intensity values in the second set are intensities of infrared, IR, light,
- generating a first spatial distribution of PPG pulse amplitudes based on the first set of light intensity values,
- generating a second spatial distribution of PPG pulse amplitudes based on the second set of light intensity values,
- comparing the first spatial distribution to the second spatial distribution, and to the location at which the substance has been applied, and
- outputting an indication of whether the subject is experiencing an allergic reaction to the substance based on the comparing.

Further fields of application are conceivable. It has been realized that remote, non-obtrusive PPG techniques are also suitably applicable in the non-medical field, e.g. in the personal care field. A main benefit of a camera-based remote PPG monitoring approach is that a considerably large area may be observed.

Reference is made again to Fig. 3. Information obtained by the image sensor 32 may be regarded as skin condition information. The skin condition information is transferred to a control unit 70. In the exemplary embodiment shown in Fig. 3, the control unit 70 is integrated in a hand-held computing device 72. The device 72 may be arranged, for instance, as a mobile phone, a mobile computer, a tablet computer, etc. Needless to say, the control unit 70, in the alternative, may also be integrated in or directly coupled to one of the hair cutting appliance 42, the illumination unit 20 and/or the sensing unit 30 (refer to Fig. 1).

In the control unit 70, a processor or processing unit 74 is provided. Further, user controls 76 are present. In addition, a display 78 is provided which may be arranged as a touch-sensitive display. Further, a speaker 80 may be provided.

The control unit 70 provides sufficient computing capacity to process the image data provided by the at least one sensor 72. In this way, skin condition information may be obtained from the skin portion 64, preferably using PPG techniques. In this way, imminent or emerging skin irritations in the skin portion 64 may be detected. Further, since the image sensor 32 is arranged to monitor a considerably large region, also the position of the hair cutting appliance 42 (with respect to the skin portion 64 of the user 62) may be detected and tracked. In this way, the position of the hair cutting appliance 42 and a "map" of the skin portion 64 of the user 62 may be correlated. In other words, one and the same information (data stream of images) may be used and processed for two analyses, i.e. position detection/tracking and skin condition detection. This is not possible with conventional contact PPG devices.

As a consequence, whenever a certain likelihood of skin irritations is detected, appropriate counter measures may be applied. To this end, the control unit 70 may be arranged to send control commands to the hair cutting appliance 42.

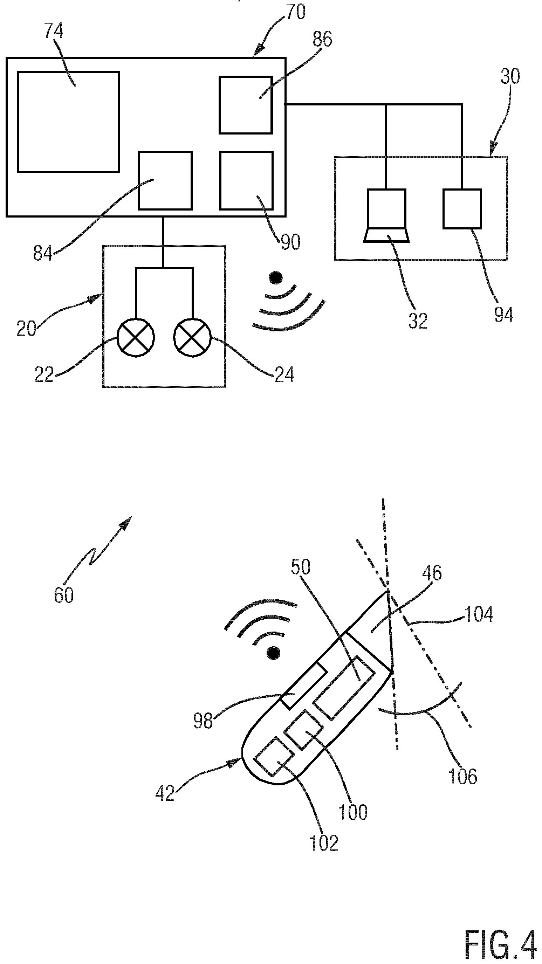

In Fig. 4, a further exemplary setting of an operation control system 60 for a personal care procedure is illustrated. As with the embodiment shown in Fig. 3, a control unit 70 is provided that is operatively coupled with a sensing unit 30 and an illumination unit 20. The illumination unit 20 comprises a first light source 22 and a second light source 24, for instance. The sensing unit 30 is provided with an image sensor 32. Further, by way of example, the sensing unit 30 may further be provided with a supplemental sensor 94. By way of example, the sensor 34 may be arranged as a position detection sensor.

In addition to the processing unit 74, the control unit 70 may be provided with an illumination control 84 and a sensor control 86. Hence, the control unit 70 is operatively coupled with the sensing unit 30 and, at least in some embodiments, with the illumination unit 20.

The control unit 70 is further equipped with a communication interface 90. Similarly, the hair cutting appliance 42 which is also illustrated in Fig. 4 may be equipped with a communication interface 98. In the exemplary embodiment of Fig. 4, the communication interfaces 90, 98 are arranged for wireless communication. Via the communication interface 90, the control unit 70 may send control commands to the hair cutting appliance 42. The control commands may initiate corrective measures when skin irritations or, at least, a certain likelihood of emerging skin irritations have/has been detected.

Hence, the control unit 70 may be operatively coupled to the operation control unit 50 of the hair cutting appliance 42. The hair cutting appliance 42 is further equipped with an indicator 100. The indicator 100 may involve a visual indicator, a sound indicator and/or a tactile indicator. Hence, having received a respective control command via the communication interface 98, the user may be prompted by the indicator 100 to decelerate the movement of the hair cutting appliance 42, for instance. In the alternative, the user may be prompted to slow an operation speed of the processing head 46 of the appliance. Further, the user may be prompted to avoid a particularly susceptible and vulnerable skin region.

The hair cutting appliance 42 may be further equipped with a position reference 102 that may be arranged as a marker. Hence, in some embodiments, when the sensing unit 30 is equipped with any of an image sensor 32 and a supplemental sensor 94, the position of the hair cutting appliance 42 may be detected and tracked using the position reference 102. Non-visual tracking (e.g. electromagnetic tracking) may be provided in addition or in the alternative to a video tracking procedure using the at least one (image) sensor 32.

In Fig. 4, there is further indicated a processing surface 104 which may be defined by the skin surface. Further, an operating angle 106 between the processing head 46 and the processing surface 104 is indicated. An appropriate corrective action in reaction to the detection of a potentially problematic skin condition may involve an adjustment of the operating angle 106, i.e. a working angle of a blade of the hair cutting appliance 42 with respect to the processing surface 104, to avoid an intense treatment. Further, as already indicated above, corrective action may also involve a reduction of a blade speed, the provision of guards, etc.

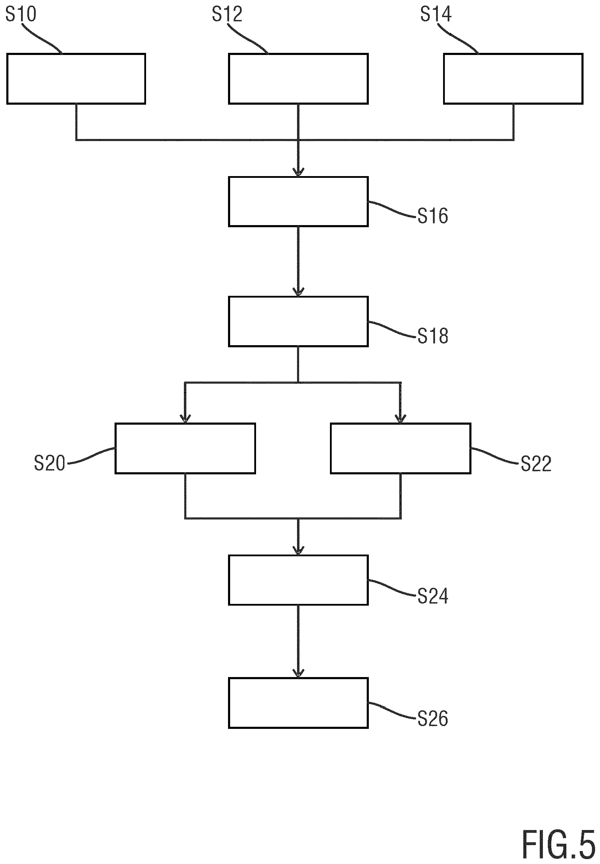

Further reference is made to Fig. 5, showing a block diagram schematically illustrating an exemplary embodiment of a method of remotely detecting a skin condition and operating a personal care appliance accordingly.

The method involves a step S10 relating to a provision of a personal care appliance for a personal care procedure. Further, provision steps S12 and S14 are provided. In a step S12, a sensing unit is provided. In a step S14, a control unit is provided. Preferably, the sensing unit S12 is arranged adjacent to or integrated in a mirror. In addition, an illumination unit may be provided that is adapted to the sensitivity of the sensing unit (not explicitly shown in Fig. 5).

Having arranged a set-up of the personal care procedure in the way, the personal care appliance may be operated, step S16. Prior to the personal care procedure, an initial detection of a skin condition of a user may be performed. In the step S16, a skin portion of the user, particularly a face portion, is engaged and processed by the personal care appliance.

When the personal care procedure is performed, images are captured by the sensing unit, step S18. Preferably, images are obtained that involve both visible and non-visible information (in terms of the spectral sensitivity of the human eye). In this way, preferably implementing remote PPG techniques, skin color information but also (blood) perfusion information that are indicative of skin conditions may be obtained. Apart from that, the data obtained in the step S18 also enables standard video processing, for instance video tracking, etc.

The data obtained in the step S18 is processed in the steps S20 and S22. The step S20 relates to the detection of skin condition-indicative information, as discussed further above. The basically parallel step S22 relates to a position detection and tracking of the personal care appliance. Hence, in a further step S24, a correlation of the position of the personal care appliance and the image information (involving a "map" of the detected skin condition properties) may be performed.

As a consequence, in a subsequent step S26, the personal care appliance may be controlled to reduce the level of skin irritations that may emerge during the personal care procedure.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A system (60) for remotely detecting a skin condition, particularly skin irritations, the system (60) comprising:
a sensing unit (30) comprising at least one image sensor (32, 34) arranged to remotely monitor a skin portion of a user (62) while performing a personal care procedure with a personal care appliance (40),
a control unit (70) arranged to process image data comprising skin condition information that is indicative of skin conditions,
wherein the image data is provided by the sensing unit (30),
wherein the control unit (70) is further arranged to correlate position data of the personal care appliance (40) with the skin condition information, and
wherein the control unit (70) is arranged to provide control commands.

2. The system (60) as claimed in claim 1, wherein the sensing unit (30) is arranged to detect and the control unit (70) is arranged to process image data in the visible range and in the non-visible range.

3. The system (60) as claimed in claim 1 or 2, wherein the sensing unit (30) and the control unit (70) form a remote photoplethysmography device, and wherein the sensing unit (30) is a camera based non-contact sensing unit.

4. The system (60) as claimed in any of claim 1 to 3, wherein the sensing unit (30) is further capable of detecting the current position of the personal care appliance (40) in an observed region.

5. The system (60) as claimed in any of claim 1 to 4, further comprising a personal care appliance (40), particularly a hand-wearable personal care appliance, wherein the control unit (70) is operatively coupled to the personal care appliance (40).

6. The system (60) as claimed in claim 5, wherein the personal care appliance (40) is a grooming appliance, particularly a hair cutting appliance (42), that is operable to contact a skin region of a user that is monitored by the sensing unit (30).

7. The system (60) as claimed in claim 5 or 6, wherein the control unit (70) is arranged to control an operation condition of the personal care appliance (40) in reaction to a detected skin condition state.

8. The system (60) as claimed in any of claims 5 to 7, wherein the control unit (70) is arranged to adapt an intensity parameter of the personal care appliance (40).

9. The system (60) as claimed in claim 8, wherein the intensity parameter is one of a blade operation speed of the personal care appliance (40) and a working angle (108) of the personal care appliance (40) with respect to the skin (64).

10. The system (60) as claimed in any of claims 1 to 9, wherein the control unit (70) is arranged to provide user feedback commands in reaction to a detected skin condition state.

11. The system (60) as claimed in any of claims 1 to 10, wherein the skin condition information is based on at least one of skin color detection and blood circulation detection.

12. The system (60) as claimed in any of claims 1 to 11, wherein the control unit (70) is further arranged to detect temporal changes of the skin condition.

13. A method of remotely detecting a skin condition, particularly skin irritations, the method comprising:
- providing (S12) a sensing unit (30) comprising at least one image sensor (32, 34) arranged to remotely monitor a skin portion of a user while performing a personal care procedure with a personal care appliance (40),
- providing (S14) a control unit (70),
- operating the control unit (70) to process image data that is indicative of skin conditions, wherein the image data is provided by the sensing unit (30),
- deriving (S20) skin condition information from the image data, and
- operating (S24) the control unit (70) to correlate position data of the personal care appliance (40) with the skin condition information.

14. A method of operating a personal care appliance (40), particularly a grooming appliance, comprising:
- detecting the condition information in accordance with the method as claimed in claim 13,
- providing (S26) control commands for the personal care appliance (40), and
- operating the personal care appliance (40) depending on the skin condition information by the control commands.

15. A computer program comprising program code means for causing a computing device to carry out the steps of the method as claimed in claim 13 when said computer program is carried out on a computing device.
